(19) Europäisches Patentamt
European Patent Office
Office européen des brevets

(11) Publication number: **0 134 601**
**B1**

(12) # EUROPEAN PATENT SPECIFICATION

(45) Date of publication of patent specification: **22.04.87**

(51) Int. Cl.$^4$: **C 07 C 51/353**, C 07 C 53/08, C 07 C 51/12

(21) Application number: **84200965.6**

(22) Date of filing: **03.07.84**

(54) **Process for the preparation of carboxylic acids.**

(30) Priority: **28.07.83 GB 8320358**

(43) Date of publication of application:
**20.03.85 Bulletin 85/12**

(45) Publication of the grant of the patent:
**22.04.87 Bulletin 87/17**

(84) Designated Contracting States:
**BE DE FR GB NL**

(58) References cited:
**EP-A-0 018 927**
**EP-A-0 060 695**
**GB-A-1 468 940**

(73) Proprietor: **SHELL INTERNATIONALE RESEARCH MAATSCHAPPIJ B.V.**
**Carel van Bylandtlaan 30**
**NL-2596 HR Den Haag (NL)**

(72) Inventor: **Drent, Eit**
**Badhuisweg 3**
**NL-1031 CM Amsterdam (NL)**

(74) Representative: **Aalbers, Onno et al**
**P.O. Box 302**
**NL-2501 CH The Hague (NL)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).



Courier Press, Leamington Spa, England.

**Description**

The present invention relates to a process for the preparation of carboxylic acids by heating a formic acid ester in the presence of carbon monoxide, a soluble palladium catalyst and a iodide and/or bromide source. The invention relates in particular to a process for the preparation of acetic acid from methyl formate.

UK Patent Specification 1,293,193 discloses a process for the preparation of a carboxylic acid by heating a formic acid ester in the presence of carbo monoxide, a catalyst comprising a metal of Group VIII or Group IIb of the Periodic Table and an amide, heterocyclic amine, nitrile or ketone as solvent. The disclosed process requires the use of high temperatures and pressures or of high amounts of catalyst. Processes in which acetic acid is produced from methyl formate in the presence of carbon monoxide at moderate reaction conditions of temperature and pressure using a rhodium catalyst are for example disclosed in UK Patent Specification 1,286,224 and US Patent Specification 4,194,056.

Because rhodium is very expensive it is attractive to develop processes in which less expensive catalysts can be used at moderate temperatures and pressures. German Offenlegungsschrift 3,046,899 discloses a process for the preparation of acetic acid in which methyl formate is isomerized at moderate conditions in the presence of carbon monoxide, a palladium, iridium or ruthenium catalyst, a halogen promotor, and optionally a secondary organic promoter such as an amine, phosphine, amide or nitrile. Palladium and ruthenium catalysts are much cheaper than rhodium catalysts.

However, for the production of carboxylic acids on a large scale it is important that even when a relatively cheap catalyst is used, the activity of the catalytic system formed in the reaction mixture by interaction of the metal compound, the halogen containing promoter and other compounds present be as high as possible. The production rate of carboxylic acid can be expressed as grams of carboxylic acid produced per gram of metal per gram of halogen present in the halogen containing promotor per hour. The production rate expressed in this way is a suitable measure for the activity of the catalytic system formed in the reaction mixture because the reaction is accelerated in proportion to both the high amount of metal catalyst and the amount of halogen containing promotor.

It has now surprisingly been found that the activity of a catalytic system based on a palladium catalyst and an iodine or bromine containing promoter can considerably be enhanced by the simultaneous presence of a nickel co-catalyst and an amide group containing compound or a sulphone.

The present invention therefore relates to a process for the preparation of a carboxylic acid of the general formula RCOOH wherein R represents an alkyl, cycloalkyl or aralkyl group by heating an ester of formic acid of the general formula HCOOR wherein R is as defined above in the presence of carbon monoxide, a soluble palladium catalyst and an iodide and/or bromide source in which a nickel co-catalyst and an organic nitrogen compound containing a group

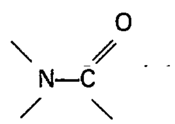

or a sulphone are present in the reaction mixture.

By means of the process of the invention production rates are attained which are very high in comparison to production rates calculated from prior art processes. Consequently smaller quantities of expensive palladium catalysts and iodide and/or bromide containing promotors, which are generally corrosive, are needed to obtain satisfactory results.

The formic acid ester used as starting material in the process according to the invention has the general formula HCOOR wherein R represents an alkyl group having preferably 1—20 and in partiuclar 1—6 carbon atoms, a cycloalkyl group having preferably 3—10 and in particular 5 or 6 carbon atoms or an aralkyl group having preferably 7—15 and in particular 7—10 carbon atoms. The process of the invention is suitable for the preparation of carboxylic acids such as for example propionic acid, n-butyric acid, iso-butyric acid, lauric acid, cyclohexyl carboxylic acid, phenylacetic acid and in particular acetic acid.

Suitable soluble palladium compounds which can be used as catalyst in the process of the invention are soluble palladium salts such as for example palladium chloride, palladium bromide, palladium chloride dihydrate, palladium iodide, palladium nitrate, palladium formate, palladium acetate, palladium propionate, palladium iso-butyrate or soluble palladium complexes such as for example palladium acetylacetonate. $Na_2PdCl_4$, $K_2PdCl_4$, $K_2PdJ_4$, $[Pd(CO)Cl_2]_2$, $Pd[(C_6H_5)_3P]_2J_2$, $[(n\text{-}C_4H_9)_4P]_2[PdCl_4]$, $Pd[(C_6H_5)_3P]_2(CO)Br$ and $Pd[(n\text{-}C_4H_9)_3P]_2J_2$.

The amount of palladium catalyst used in the process of the invention is not critical and any amount which exerts catalytic activity can be used. The catalyst is preferably used in an amount of $10^{-2}$—$10^{-6}$ and in particular of $10^{-3}$—$10^{-5}$ gram-atoms, based on palladium, per mol formic acid ester.

The iodide and/or bromide source used in the process of the invention may be for example elemental iodine or bromine, or hydrogen iodide or bromide, or an alkali metal or an alkaline earth metal iodide or bromide such as for example lithium iodide, sodium iodide, potassium iodide, rubidium iodide, cesium iodide, lithium bromide, sodium bromide, potassium bromide, rubidium bromide, cesium bromide,

calcium iodide, calcium bromide, magnesium iodide, magnesium bromide, or an ammonium, phosphonium, arsonium or stibonium iodide or bromide such as for example triphenylphosphonium iodide, methyltriphenyl phosphonium iodide, tetramethylammonium iodide and tetrabutylammonium iodide. Further bromide and/or iodide sources are organic compounds of general formula $R^1(CO)nHal$ wherein n=0 or 1, Hal represents Br or J and $R^1$ represents an alkyl group having up to 1—12 carbon atoms or an aryl, aralkyl or alkaryl group having up to 12 carbon atoms such as for example $CH_3Br$, $C_2H_5J$, $C_4H_9J$, $C_8H_{17}J$ and in particular $CH_3J$. The use of an alkali metal iodide or a mixture of an alkali metal iodide and an alkyl iodide, in particular $CH_3J$, is preferred.

The total amount of the iodide and/or bromide source present in the reaction mixture is equivalent to 1—1000, preferably 3—500 and in particular 10—300 gram-atoms iodine and/or bromine per gram-atom palladium.

Suitable nickel co-catalysts which can be used in the process of the present invention are for example soluble nickel compounds such as nickel chloride, nickel chloride hexahydrate, nickel iodide, nickel nitrate or nickel acetate or nickel complexes such as for example bishalobisphosphino or bissalicylaldiminato complexes of nickel.

The amount of nickel compound used is generally equivalent to 2—100, preferably 5—70 and in particular 10—50 gram-atoms nickel per gram atom palladium.

The organic nitrogen compound containing a group

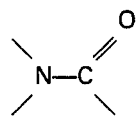

or the sulphone used in the process of the invention forms a solution with the reacting materials and/or formed products under the reaction conditions. Very suitable organic nitrogen compounds containing a group

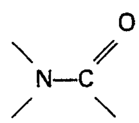

are amides, carbamates urea and urea derivatives which are fluids at the temperatures and pressures applied during the reaction. Suitable amides are for example amides of the general formula

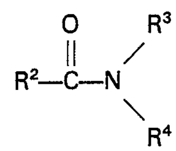

wherein $R^2$ represents H or an alkyl, cycloalkyl, aryl, alkaryl or aralkyl group having up to 10 carbon atoms, $R^3$ and $R^4$ each represent H or an alkyl, aryl, alkaryl or aralkyl group, which may contain a group

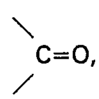

having up to 10 carbon atoms, or $R^3$ and $R^4$ together with the nitrogen atom form a cyclic group having up to 5 carbon atoms or $R^2$ and $R^3$ or $R^4$ together with the nitrogen atom form a cyclic group having up to 5 carbon atoms. Specific examples of these amides are formamide, acetamide, N-methylacetamide, N-ethylacetamide, N,N-dimethylformamide, N,N-diethylformamide, N,N-dimethylacetamide, N,N-diethylacetamide, N-methyl-N-ethylacetamide, N,N - dimethylcyclohexyl carboxamide, N,N - dimethylbenzamide, N - phenylacetamide, N,N - diphenylacetamide, N - methylpyrrolidone, N - formylmorpholine, N-acetylmorpholine, N-formylpiperidine, N-acetylpiperidine and N-acetyl-N'-methylpiperazine. Especially suitable are N,N - dimethylacetamide and N - methylpyrrolidone. Amides of the above general formula also include diamides, triamides such as diacetamide, triacetamide, dibenzamide, tribenzamide and N-methyldibenzamide and imides such as succinimide, 1,2 cyclohexane dicarboximide and N-phenylphthalimide.

Suitable carbamates are for example carbamates of the general formula

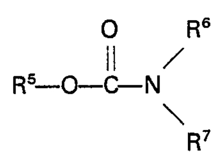

wherein $R^5$ represents an alkyl, aryl, aralkyl or alkaryl group having up to 10 carbon atoms and $R^6$ and $R^7$ each repsent H or an alkyl, aryl, aralkyl or alkaryl group having up to 10 carbon atoms. Specific examples of these carbamates are methyl carbamate, ethyl carbamate, phenyl carbamate, methyl N-methylcarbamate, methyl N-ethylcarbamate, methyl N-phenylcarbamate, ethyl N-methylcarbamate, ethyl N-ethylcarbamate, phenyl N-ethylcarbamate, phenyl N-phenylcarbamate, methyl N,N-dimethylcarbamate, methyl N,N-diethylcarbamate, methyl N,N - diphenylcarbamate, ethyl N,N - dimethylcarbamate, ethyl N,N - diethylcarbamate, ethyl N,N-diphenylcarbamate, phenyl N,N-dimethylcarbamate, phenyl N,N-diethylcarbamate, phenyl N,N-diphenylcarbamate, methyl N - methyl - N - ethylcarbamate and ethyl N - methy - N - ethylcarbamate. Urea and urea derivatives which can be used in the process of the invention have the general formula

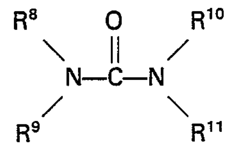

wherein $R^8$, $R^9$, $R^{10}$ and $R^{11}$ each represent H or an alkyl, aryl, aralkyl, or alkaryl group having up to 10 carbon atoms. Specific examples of these compounds are urea, 1,3-dimethylurea, 1,3-diethylurea, 1,3-diphenylurea, 1,1 - dimethylurea, 1,1 - diphenylurea and 1,1,3,3 - tetramethylurea. Suitable sulphones are for example sulphones of the general formula

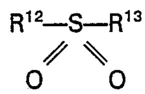

wherein $R^{12}$ and $R^{13}$ each represent an alkyl, aryl, aralkyl or alkaryl group having up to 10 carbon atoms or $R^{12}$ and $R^{13}$ together with the sulfur atom form a cyclic group having up to 5 carbon atoms. Specific examples of these sulphones are dimethyl sulphone, diethyl sulphone, methylethyl sulphone, methylbutyl sulphone, butylphenyl sulphone, dibenzyl sulphone, 4,4'-ditolyl sulphone, 4,4'-diacetamidodiphenyl sulphone, sulpholane, 2-methyl sulpholane, 3-methyl sulpholane or 2-methyl-4-butyl sulpholane. The use of sulpholane or a methyl substituted sulpholane is preferred.

In general the organic nitrogen compound containing a group

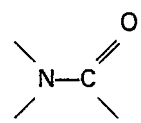

or the sulphone is present in the reaction mixture in an amount of at least 0.01 mol per mol formic acid ester and preferably in an amount in the range of 0.05—3 mol per mol formic acid ester.

If desired, the process according to the invention may be carried out in the presence of an additional promotor. Suitable additional promotors are orgnaic phosphorus compounds represented by the general formula

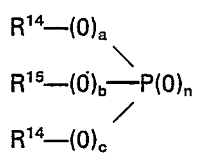

in which n is 0 or 1, either a, b and c are 0 or 1 and $R^{14}$, $R^{15}$ and $R^{16}$ are similar or dissimilar optionally substituted hydrocarbon groups, or a and b are O and c is 0 or 1 and $R^{14}$ and $R^{15}$ form together with P a heterocyclic group. Preferance is given to compounds represented by the above general formula in which a, b, and c are 0. In these compounds the group $R^{14}$, $R^{15}$ and $R^{16}$ are preferably similar or dissimilar alkyl groups containing 1—12 carbon atoms or cycloalkyl, aryl or alkaryl groups containing 5—12 carbon atoms optionally substituted with groups which are substantially inert in the reaction medium such as chlorine, alkoxy groups, carboxylic ester groups or oxo groups.

Very suitable additional promotors are tertiary phosphines and the oxides thereof such as tri-n-butylphosphine, tri-n-butylphosphine oxide, triethylphosphine, triethylphosphine oxide, tricyclohexylphosphine, tricyclohexylphosphine oxide, triphenylphosphine, triphenylphosphine oxide, tri-p-tolylphosphine oxide, tri - p - chlorophenylphosphine and l - phenylphospholane oxide. Most preferred are triphenylphosphine and triphenylphosphine oxide.

The amount of additional promotor which can be used in the process of the invention is not critical and may be in the range of from 0.01 to 200 mols organic phosphorus compound per gram-atom palladium.

It will be appreciated that in the reaction mixture salts or complexes may be formed by the reaction of the organic phosphorus compounds with the iodine or bromine compound(s) present. An example of such a complex is $[(C_6H_5)_3PO\text{-}H\text{-}OP(C_6H_5)_3]^+J_3^-$. Consequently the use of such salts or complexes as additional promotors when prepared separately is within the scope of the present invention. Furthermore it will be appreciated that the oxides of the phosphines can be formed in situ from the corresponding phosphines by carrying out the reaction in the presence of molecular oxygen or hydrogen peroxide.

The process according to the present invention can be carried out using a temperature in the range of 50°C to 250°C. Preference is given to a temperature in the range of 110°C to 225°C and particularly in the range of 125°C to 200°C. In general the reaction is carried out at a partial pressure of carbon monoxide of 0.5 bar to 70 bar. Higher pressures as high as 1000 bar can be applied, but they are generally not economical because of the investments and energy costs involved. Partial pressures of carbon monoxide in the range of 10 bar to 60 bar are preferred.

The carbon monoxide used in the process of the invention may contain inert gases such as for example nitrogen, noble gases, carbon dioxide or methane. Mixtures of carbon monoxide and hydrogen i.e. synthesis gas can also be used. Synthesis gas has the advantage that it is easily available. The molar ratio of hydrogen to carbon monoxide may be in the range of 0 to 3 and preferably in the range of 0 to 1.

The process of the invention is carried out in the liquid phase. Usually there is no need for the use of an (additional) solvent since the formic acid ester used as starting material and/or the formed carboxylic acid have sufficient degree of solvent activity. Other components of the reaction mixture, for example a liquid iodide source or a liquid phosphine (oxide) may also be active as solvents. If desired, additional quantities of these compounds may be added to the reaction mixture. Suitable (additional) solvents are preferably carboxylic acids or esters such as for example, acetic acid, propionic acid, butyric acid, lauric acid, methyl acetate or butyrolactone.

The process of the invention is preferably carried out under anhydrous conditions. Small quantites of water such as present in commercially available starting materials can be allowed. The process according to the invention may be carried out continuously or batch-wise. The reaction mixture obtained may be worked-up with the aid of known techniques, such as fractional distillation. Furthermore, the process may be integrated into existing processes for preparing the starting material or for further processing the carboxylic acid obtained.

Example I

A magnetically stirred 300-ml Hastelloy C autoclave (Hastelloy is a trade mark) was charged with 35 ml methyl formate, 35 ml N - methylpyrrolidone, 0.1 mmol palladium acetate, 2.4 mmol $NiCl_2\text{-}6H_2O$ and quantities of further ingredients as indicated in Table A. The vessel was flushed with carbon monoxide and then pressurized with a mixture of carbon monoxide and hydrogen to 45 bar (partial pressure of carbon monoxide 30 bar, partial present of hydrogen 15 bar). The autoclave was then heated to 170°C and kept at this temperature during 3 hours. After the reaction time the reaction mixture was cooled and analyzed by gas-liquid chromatography. The production rate of acetic acid was expressed as grams of acetic acid per gram palladium per gram iodine per hour.

When experiment 3 was repeated with either pyridine or diglyme as solvent instead of N-methylpyrrolidone and 10 ml methyl formate instead of 35 ml methyl formate, only traces of acetic acid were produced. When experiment 2 was repeated except that no nickel co-catalyst was present, the production rate of acetic acid was 160 gr acetic acid per gram palladium per gram iodine per hour.

Example II

A magnetically stirred 300-ml Hastelloy C autoclave (Hastelloy is a trade mark) was charged with 10 ml methyl formate, 35 ml sulpholane and quantites of further ingredients as indicated in Table B. The vessel was flushed with carbon monoxide and then pressurized with a mixture of carbon monoxide and hydrogen to 45 bar. (partial pressure of carbon monoxide 30 bar, partial pressure of hydrogen 15 bar). The autoclave was then heated to 170°C and kept at this temperature during a number of hours as indicated in Table B. The further procedure was as described in Example I. The comparative experiments 2 and 3 show that by using either a palladium catalyst or a nickel inferior results are obtained.

TABLE A

| Experiment nr. | Methyl iodide mmol | Alkali. metal iodine mmol | Triphenyl phosphine oxide mmol | Production rate g acetic acid/gr Pd /gr J/h |
|---|---|---|---|---|
| 1 | 12.5 | LiJ 18 | | 210 |
| 2 | 12.5 | LiJ 18 | 12 | 227 |
| 3 | | KJ 18 | 12 | 362 |

TABLE B

| Experiment nr. | Palladium acetate mmol | $NiCl_2$-$6H_2O$ mmol | Methyl iodine mmol | Alkali metal iodine mmol | Triphenyl phosphine oxide mmol | Reaction time h | Production rate g acetic acid/ g Pd/g J/h |
|---|---|---|---|---|---|---|---|
| 1 | 0.1 | 2.4 | | KJ 18 | 12 | 1 | 199 |
| 2 | 0.1 | | 18 | LiJ 12 | 12 | 2.5 | 46 |
| 3 | | 2.4 | 18 | LiJ 12 | 12 | 3 | 3* |

*calculated on Ni.

**Claims**

1. A process for the preparation of a carboxylic acid of the general formula RCOOH wherein R represents an alkyl, cycloalkyl, or aralkyl group by heating an ester of formic acid of general formula HCOOR wherein R is as defined above in the presence of carbon monoxide, a soluble palladium catalyst and an iodide and/or bromide source characterized in that a nickel co-catalyst and an organic nitrogen compound containing a group

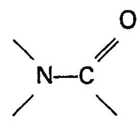

or a sulphone are present in the reaction mixture.

2. A process according to claim 1 characterized in that the organic nitrogen compound containing a group

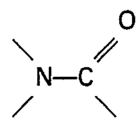

or the sulphone is present in an amount of at least 0.01 mol per mol formic acid ester and preferably in an amount in the range of 0.05-3 mols per mol formic acid ester.

3. A process according to claim 1 or 2 characterized in that the organic nitrogen compound containing a group

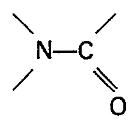

is an amide of the general formula

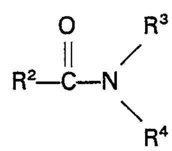

wherein $R^2$ represents H or an alkyl, cycloalkyl, aryl, alkaryl or aralkyl group having up to 10 carbon atoms, $R^3$ and $R^4$ each represent H or an alkyl, aryl, alkaryl or aralkyl group which may contain a group

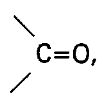

having up to 10 carbon atoms, or $R^3$ and $R^4$ together with the nitrogen atom form a cyclic group having up to 5 carbon atoms or $R^2$ and $R^3$ or $R^4$ together with the nitrogen atom form a cyclic group having up to 5 carbon atoms.

4. A process according to any one of claims 1—3 characterized in that the amide is N-methylpyrrolidone.

5. A process according to claim 1 or 2 characterized in that the organic nitrogen compund containing a group

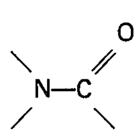

is a carbamate of the general formula

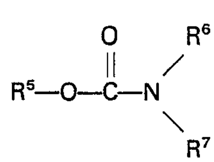

wherein $R^5$ represents an alkyl, aryl, aralkyl or alkaryl group having up to 10 carbon atoms and $R^6$ and $R^7$ each represent H or an alkyl, aryl, aralkyl or alkaryl group having up to 10 carbon atoms.

6. A process according to claim 1 or 2 characterized in that the organic nitrogen compound containing a group

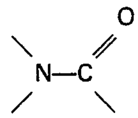

is urea or a urea derivative of the general formula

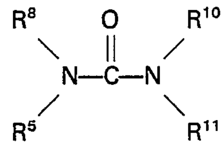

wherein $R^8$, $R^9$, $R^{10}$ and $R^{11}$ each represent H or an alkyl, aryl, aralkyl or alkaryl group having up to 10 carbon atoms.

7. A process according to claim 1 or 2 characterized in that the sulphone is a sulphone of the general formula

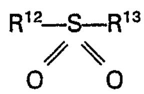

wherein $R^{12}$ and $R^{13}$ each represent an alkyl, aryl, aralkyl or alkaryl group having up to 10 carbon atoms or $R^{12}$ and $R^{13}$ together with the sulfur atom form a cyclic group having up to 5 carbon atoms.

8. A process according to any one of claims 1, 2 or 7 characterized in that the sulphone is sulpholane or a methyl substituted sulpholane.

9. A process according to any one of claims 1—8 characterized in that the iodide and/or bromide source is elemental iodine or bromine, hydrogen iodide or bromide, an alkali metal iodide or bromide, an alkaline earth metal iodide or bromide or an organic compound having general formula $R^1(CO)_n$ Hal wherein N=0 or 1, Hal represents Br or J and $R^1$ represents an alkyl group having 1—12 carbon atoms or an aryl, aralkyl or alkaryl group having up to 12 carbon atoms.

10. A process according to any one of claims 1—9 characterized in that the iodide and/or bromide source is an alkali metal iodide or a mixture of an alkali metal iodide and an alkyl iodide.

11. A process according to any one of claims 1—10 characterized in that the iodide and/or bromide source is present in a total amount equivalent to 1—1000, preferably 3—500 and in particular 10—300 gram-atoms iodine and/or bromine per gram-atom palladium.

12. A process according to any one of claims 1—10 characterized in that a tertiary phosphine or an oxide thereof is present as additional promotor.

13. A process according to any one of claims 1—12 characterized in that the additional promotor is triphenylphosphine or triphenylphosphine oxide.

14. A process according to any one of claims 1—13 characterized in that the reaction is carried out at a partial pressure of carbon monoxide of 0.5 bar to 70 bar and preferably 10 bar to 60 bar.

**Patentansprüche**

1. Ein Verfahren zur Herstellung einer Carbonsäure der allgemeinen Formel RCOOH, in der R eine Alkyl-, Cycloalkyl- oder Aralkylgruppe darstellt, durch Erhitzen eines Esters der Ameisensäure der allgemeinen Formel HCOOR, in der R wie vorstehend definiert ist, in Gegenwart von Kohlenmonoxid, eines löslichen Palladiumkatalysators und einer Jodid- und/oder Bromidquelle, dadurch gekennzeichnet, daß in dem Reaktionsgemisch ein Nickel-Cokatalysator und eine organische Stickstoffverbindung, die eine Gruppe

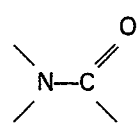

enthält, oder ein Sulfon vorhanden sind.

2. Ein Verfahren nach Anspruch 1, dadurch gekennzeichnet, daß die organische Stickstoffverbindung, die eine Gruppe

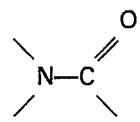

enthält, oder das Sulfon in einer Menge von mindestens 0,01 Mol je Mol Ameisensäureester und vorzugswise in einer Menge im Bereich von 0,05—3 Mol je Mol Ameinsensäureester vorhanden ist.

3. Ein Verfahren nach Anspruch 1 oder 2, dadurch gekennzeichnet, daß die organische Stickstoffverbindung, die eine Gruppe

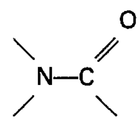

enthält, ein Amid der Allgemeinen Formel

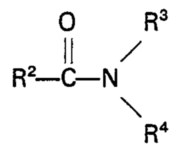

ist, in der $R^2$ Wasserstoff der eine Alkyl-, Cycloalkyl-, Aryl- Alkaryl- oder Aralkylgruppe mit bis zu 10 Kohlenstoffatomen darstellt, $R^3$ und $R^4$ jeweils Wasserstoff oder eine Alkyl-, Aryl-, Alkaryl- oder Aralkylgruppe, die eine Gruppe

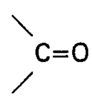

enthalten kann, mit bis zu 10 Kohlenstoffatomen darstellen oder $R^3$ und $R^4$ zusammen mit dem Stickstoffatom eine cyclische Gruppe mit bis zu 5 Kohlenstoffatomen bilden oder $R^2$ und $R^3$ oder $R^4$ zusammen mit dem Stickstoffatom eine cyclische Gruppe mit bis zu 5 Kohlenstoffatomen bilden.

4. Ein Verfahren nach irgendeinem der Ansprüche 1—3, dadurch gekennzeichnet, daß das Amid N-Methylpyrrolidon ist.

5. Ein Verfahren nach Anspruch 1 oder 2, dadurch gekennzeichnet, daß die organische Stickstoffverbindung, die eine Gruppe

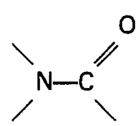

enthält, ein Carbamat der allgemeinen Formel

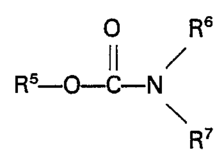

ist, in der $R^5$ eine Alkyl-, Aryl-, Aralkyl- oder Alkaryl- gruppe mit bis zu 10 Kohlenstoffatomen darstellt und $R^6$ und $R^7$ jeweils Wasserstoff oder eine Alkyl-, Aryl-, Aralkyl- oder Alkarylgruppe mit bis zu 10 Kohlenstoffatomen darstellen.

6. Ein Verfahren nach Anspruch 1 oder 2, dadurch gekennzeichnet, daß die organische Stickstoffverbindung, die eine Gruppe

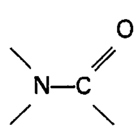

enthält, Harnstoff oder eine Harnstoff-Derivat der allgemeinen Formel

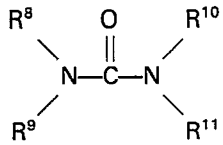

ist, in der $R^8$, $R^9$, $R^{10}$ und $R^{11}$ jeweils Wasserstoff oder eine Alkyl-, Aryl-, Aralkyl- oder Alkarylgruppe mit bis zu 10 Kohlenstoffatomen darstellen.

7. Ein Verfahren nach Anspruch 1 oder 2, dadurch gekennzeichnet, daß das Sulfon ein Sulfon der allgemeinen Formel

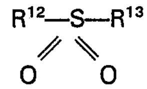

ist, in der $R^{12}$ und $R^{13}$ jeweils eine Alkyl-, Aryl-, Aralkyl- oder Alkarylgruppe mit bis zu 10 Kohlenstoffatomen darstellen oder $R^{12}$ und $R^{13}$ zusammen mit dem Schwefelatom eine cyclische Gruppe mit bis zu 5 Kohlenstoffatomen bilden.

8. Ein Verfahren nach irgendeinem der Ansprüche 1, 2 oder 7, dadurch gekennzeichnet, daß das Sulfon Sulfolan oder ein methylsubstituiertes Sulfolan ist.

9. Ein Verfahren nach irgendeinem der Ansprüche 1—8, dadurch gekennzeichnet, daß die Jodid- und/oder Bromidquelle elementares Jod oder Brom, Jod- oder Bromwasserstoff, ein Alkalimetalljodid oder -bromid, ein Erdalkalimetalljodid oder -bromid oder eine organische Verbindung mit der allgemeinen Formel $R^1$ $(CO)_nHal$ ist, in der n=0 oder 1 ist, Hal Brom oder Jod darstellt und $R^1$ eine Alkylgruppe mit 1—12 Kohlenstoffatomen oder eine Aryl-, Aralkyl- oder Alkarylgruppe mit bis zu 12 Kohlenstoffatomen darstellt.

10. Ein Verfahren nach irgendinem der Ansprüche 1—9, dadurch gekennzeichnet, daß die Jodid- und/oder Bromidquelle ein Akalimetalljdodi oder ein Gemisch eines Alkalimetalljodids und eines Alkyljodids ist.

11. Ein Verfahren nach irgendeinem der Ansprüche 1—10, dadurch gekennzeichnet, daß die Jodid- und/oder Bromidquelle in einer Gesamtmenge vorhanden ist, die äquivalent zu 1—1000, vorzugsweise 3—500 und insbesondere 10—300 Grammatome Jod und/oder Brom je Grammatom Palladium ist.

12. Ein Verfahren nach irgendeinem der Ansprüche 1—10, dadurch gekennzeichnet, daß ein tertiäres Phosphin oder dessen Oxid als zusätzlicher Promotor vorhanden ist.

13. Ein Verfahren nach irgendeinem der Ansprüche 1—12, dadurch gekennzeichnet, daß der zusätzliche Promotor Triphenylphosphin oder Triphenylphosphinoxid ist.

14. Ein Verfahren nach irgendeinem der Ansprüche 1—13, dadurch gekennzeichnet, daß die Reaktion bei einem Kohlenmonoxidpartialdruck von 0,5 bis 70 bar und vorzugsweise 10 bar bis 60 bar durchgeführt wird.

**Revendications**

1. Un procédé pour la préparation d'un acide carboxylique de la formule générale RCOOH où R représente un groupe alcoyle, cycloalcoyle ou aralcoyle par chauffage d'un ester de l'acide formique de la formule générale HCOOR où R est tel que défini ci-dessus en présence d'oxyde de carbone, d'un catalyseur au palladium soluble et d'une source de bromure et/ou d'iodure, caractérisé en ce qu'un cocatalyseur au nickel et un composé organique azoté contenant un group

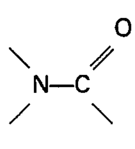

ou une sulfone sont présents dans le mélange réactionnel.

2. Un procédé selon la revendication 1, caractérisé en ce que le composé organique azoté contenant un groupe

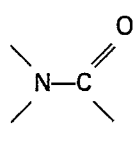

ou la sulfone est présent à raison d'au moins 0,01 mole par mole d'ester d'acide formique et de préférence à raison de 0,05 à 3 moles par mole d'ester d'acide formique.

3. Un procédé selon la revendication 1 ou 2, caractérisé en ce que le composé organique azoté contenant un groupe

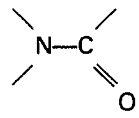

est un amide de la formule générale

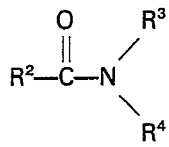

dans laquelle $R^2$ représente H ou un groupe alcoyle, cycloalcoyle, aryle, alcaryle ou aralcoyle ayant jusqu'à 10 atomes de carbone, $R^3$ et $R^4$ représentent chacun H ou un groupe alcoyle, aryle, alcaryle ou aralcoyle qui peut contenir un groupe

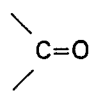

ayant jusqu'à 10 atomes de carbone, ou $R^3$ et $R^4$ forment avec l'atome d'azote un groupe cyclique ayant jusqu'à 5 atomes de carbone ou $R^2$, $R^3$ et $R^4$ forment avec l'atome d'azote un groupe cyclique ayant jusqu'à 5 atomes de carbone.

4. Un procédé selon l'une quelconque des revendications 1—3, caractérisé en ce que l'amide est la N-méthylpyrrolidone.

5. Un procédé selon la revendication 1 ou 2, caractérisé en ce que le composé organique azoté contenant un groupe

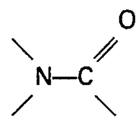

est un carbamate de la formule générale

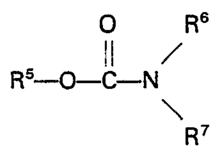

où $R^5$ représente un groupe alcoyle, aryle, aralcoyle ou alcaryle ayant jusqu'à 10 atomes de carbone et $R^6$ et $R^7$ représentant chacun H ou un groupe alcoyle, aryle, aralcoyle ou alcaryle ayant jusqu'à 10 atomes de carbone.

6. Un procédé selon la revendication 1 ou 2, caractérisé en ce que le composé organique azoté contenant un groupe

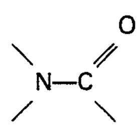

est de l'urée ou un dérivé de l'urée de la formule générale

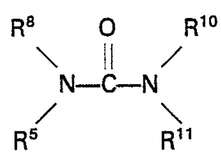

où $R^8$, $R^9$, $R^{10}$ et $R^{11}$ représentant chacun H ou un groupe alcoyle, aryle, aralcoyle ou alcaryle ayant jusqu'à 10 atomes de carbone.

7. Un procédé selon la revendication 1 ou 2, caractérisé en ce que la sulfone est une sulfone de la formule générale

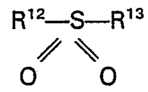

où $R^{12}$ et $R^{13}$ représentent chacun un groupe alcoyle, aryle, aralcoyle ou alcaryle ayant jusqu'à 10 atomes de carbone ou $R^{12}$ et $R^{13}$ forment avec l'atome de soufre un groupe cyclique ayant jusqu'à 5 atomes de carbone.

8. Un procédé selon l'une quelconque des revendications 1, 2 ou 7, caractérisé en ce que la sulfone est du sulfolane ou un méthyl sulfolane.

9. Un procédé selon l'une quelconque des revendications 1—8, caractérisé en ce que la source d'iodure et/ou de bromure est de l'iode ou du brome élémentaire, de l'iodure ou du bromure d'hydrogène, un iodure ou bromure de métal alcalin, un iodure ou bromure de métal alcalinoterreux ou un composé organique ayant la formule générale $R^1(CO)_nHal$ dans laquelle n=0 ou 1, Hal représente Br ou I et $R^1$ représente un groupe alcoyle ayant 1—12 atomes de carbone ou un groupe aryle, aralcoyle ou alcaryle ayant jusqu'à 12 atomes de carbone.

10. Un procédé selon l'une quelconque des revendications 1—9, caractérisé en ce que la source d'iodure et/ou de bromure est un iodure de métal alcalin ou un mélange d'un iodure de métal alcalin et d'un iodure d'alcoyle.

11. Un procédé selon l'une quelconque des revendications 1—10, caractérisé en ce que la source d'iodure et/ou de bromure est présente dans une quantité totale équivalente à 1—1000, de préférence 3—500 et en particulier 10—300 atomes-grammes d'iode et/ou de brome par atome-gramme de palladium.

12. Un procédé selon l'une quelconque des revendications 1—10, caractérisé en ce qu'une phosphine tertiaire ou un oxyde d'un tel composé est présent comme promoteur supplémentaire.

13. Un procédé selon l'une quelconque des revendications 1—12, caractérisé en ce que le promoteur supplémentaire est de la triphénylphosphine ou de l'oxyde de triphénylphosphine.

14. Un procédé selon l'une quelconque des revendications 1—13, caractérisé en ce que la réaction est conduite à une pression partielle d'oxyde de carbone de 0,5 à 70 bars et de préférence de 10 à 60 bars.